(19) Europäisches Patentamt
European Patent Office
Office européen des brevets

(11) **EP 3 906 856 A1**

(12) **EUROPEAN PATENT APPLICATION**

(43) Date of publication:
**10.11.2021 Bulletin 2021/45**

(51) Int Cl.:
**A61B 6/00** *(2006.01)*        **H01J 35/08** *(2006.01)*

(21) Application number: **20173258.3**

(22) Date of filing: **06.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Excillum AB**
**164 40 Kista (SE)**

(72) Inventors:
- **Hansson, Björn**
  **164 40 Kista (SE)**
- **Hållstedt, Julius**
  **164 40 Kista (SE)**
- **Tuohimaa, Tomi**
  **164 40 Kista (SE)**
- **Lundström, Ulf**
  **164 40 Kista (SE)**

(74) Representative: **AWA Sweden AB**
**P.O. Box 45086**
**104 30 Stockholm (SE)**

(54) **X-RAY IMAGING SYSTEM**

(57) There is disclosed an X-ray imaging system, comprising a target; an electron beam source configured to provide an electron beam for interaction with the target to generate X-ray radiation; electron optics configured to alternately direct the electron beam to at least a first and a second location on the target; an X-ray detector array configured to receive X-ray radiation generated at the first and second locations on the target; a sample position region for receiving a sample to be exposed to generated X-ray radiation, the sample position region being located in a region where X-ray radiation generated at the first location overlaps with X-ray radiation generated at the second location; and a processing unit coupled to the X-ray detector array, the processing unit being configured to create an image of a sample, positioned in the sample position region, based on the X-ray radiation originating from the first location and from the second location. In embodiments, the electron beam is moved between the locations at a sufficiently short time period such that thermal cycling, and thereby thermally induced mechanical stress, is reduced. A corresponding method is also disclosed.

*Fig. 2*

**Description**

Field of the invention

The present invention relates to an X-ray imaging system in which an electron beam interacts with a target to generate X-ray radiation.

Background

A prior art X-ray system utilizing multiple spots from which X-ray radiation is generated by interaction between an electron beam and a target is disclosed in US 5,835,561. In that system, each spot is associated with one of a plurality of collimating elements. The collimating elements are placed in front of the emitting face of the X-ray tube and each collimating element has its aperture axis directed towards the center of a detector array and provides a divergence such that the detector array is just covered. A plurality of pencil beams, one from each collimating aperture, can thus be generated each passing a different portion of the object to be imaged. When the electron beam is scanned across the target, at any given moment there is only a single X-ray pencil beam which passes through the object to the detector array. The number of collimating apertures (corresponding to the number of spots from which X-ray radiation is generated) typically corresponds to the number of pixels in the reconstructed image. The purpose of this prior art system is to provide real-time X-ray imaging of a cardiology patient at a lower radiation exposure than yet earlier systems. While such system can be useful in situations where it is desired to keep X-ray exposure low, e.g. when imaging living bodies, it is less advantageous in situations where a high X-ray power is sought.

In "Scanning-beam digital X-ray (SBDX) system for cardiac angiography" (Proc. SPIE 3659, Medical Imaging 1999; Physics of Medical Imaging, 28 May 1999), authors Solomon *et al.* disclose an advanced scanning-beam digital X-ray system for cardiac angiography. An electron beam is scanned across a transmission target that is located behind a focused source collimator. The collimator is a grid of apertures whose axes are aligned with the center of a detector array and X-ray beam divergence through the collimator apertures is matched to the detector size.

In "Motionless electromagnetic phase stepping versus mechanical phase stepping in x-ray phase-contrast imaging with a compact source" (Phys. Med. Biol. 60 (2015), p. 3031-3043), authors Harmon *et al.* disclose X-ray phase contrast imaging and mention that the X-ray focal spot can be shifted by a magnetic field in order to move the spot in a direction perpendicular or parallel, depending on the direction of the magnetic field, to the grating lines.

Summary

The present invention is based on the recognition that an improved X-ray imaging system can be obtained if an electron beam is alternately directed to different locations on a target, and a detector array for detecting generated X-ray radiation is configured to perform time resolved photon counting such that photons that are detected during a particular time frame can be attributed to a respective particular location on the target. Thereby, embodiments of the invention can provide higher photon flux without increasing the local thermal load of the target or increasing the spot size..

By ensuring that the electron beam is directed to each respective location for a duration of time that is sufficiently short for thermal equilibrium not to be established, a higher power than what could continuously be deposited at any particular location on the target may be applied. In other words, a period of continuous exposure of any one location on the target should have a duration that is shorter than a time limit.

By further ensuring that the electron beam returns, or at least approximately returns, to a particular location on the target within a sufficiently short time in order for that location not to have cooled significantly since the electron beam was last directed to that location, excessive thermal cycling of the target is avoided and problems related to thermally induced mechanical stress are avoided. In order to determine within what time period the electron beam should return to the same location (i.e. what is considered as a sufficiently short time), a characteristic time scale for each situation can be determined. In other words, a time elapsed between consecutive periods during which the electron beam is directed to each of the first and the second location on the target, respectively, should be shorter than a time limit.

Alternatively, rather than alternately moving the same electron beam between different locations, two separate electron beams can be used that are alternately switched on and off.

The target can be a solid target or a liquid target. A target can be implemented as a transmission target or as a reflection target. In embodiments where the target is implemented as a liquid target, the target is preferably one or more liquid jets. If a single liquid jet is used as the target, then the different locations between which the electron beam is alternately moved are on that liquid jet. A single liquid jet can be implemented as a flat jet used in transmission mode where the electron beam is moved between different locations along a direction substantially perpendicular to a travel direction of the liquid jet. If two or more liquid jets are used as the target, then the different locations between which the electron beam is alternately moved could be on different liquid jets. A solid reflection target may be implemented as a rotating target, i.e. a rotating anode, in such a case the electron beam may be moved between different locations along a direction substantially perpendicular to a direction of rotation of the target.

When the X-ray radiation is detected in a time-resolved fashion, each image captured by the detector can be associated with a respective one of the different locations on the target. The images associated with the different locations may then be combined into a single image of an area where the X-ray radiation from the different locations overlap, while maintaining the resolution and sharpness obtained from a single X-ray spot. Hence, at least a first and a second image of the same portion of an object are captured, wherein the first image is captured using X-ray radiation from a first location on the target and the second image is captured using X-ray radiation from a second location on the target. The images are subsequently combined in post processing into a single image of the portion of the object.

In embodiments of the invention using a solid target, the electron beam is preferably alternated between different locations on the target at a sufficiently high rate (short time period) so as to emulate a thermal steady state in the target. Thereby, mechanical stress due to thermal cycling is reduced, which in turn may lead to increased service life for the target. In other words, the electron beam is alternated between the different locations at such high rate that any temperature variation during the duty cycle can be neglected. Hence, embodiments of the invention allow an increased power of the electron beam, and thereby of generated X-ray radiation, while at the same time potential problems associated with thermal stress due to temperature cycling of the target material are mitigated.

Technology for moving an electron beam between different positions is generally known within the art, e.g. using electrostatic deflection. The electron beam can be moved between different positions at very high rates using existing technology. The upper limit for how fast the electron beam can be alternated between different locations on the target is thus not determined by the available technology for moving the electron beam, but rather by the time resolution of the detector since photons generated from each respective location should be binned together in order to allow the captured images to be combined into a composite image during post processing reconstruction.

In the detailed description below, expressions for a characteristic time scale describing generation and dissipation of heat in the target are derived. Preferably, the electron beam is moved between locations on the target with a time period less than 10 times such characteristic time scale, more preferably less than 5 times such characteristic time scale.

## Brief description of drawings

The present invention will now be described in more detail with reference to the accompanying drawings, on which

Fig. 1 illustrates a typical set-up according to one embodiment of the invention;
Fig. 2 schematically shows an X-ray imaging system according to an embodiment; and
Fig. 3 schematically illustrates a method according to the present invention.

## Detailed description

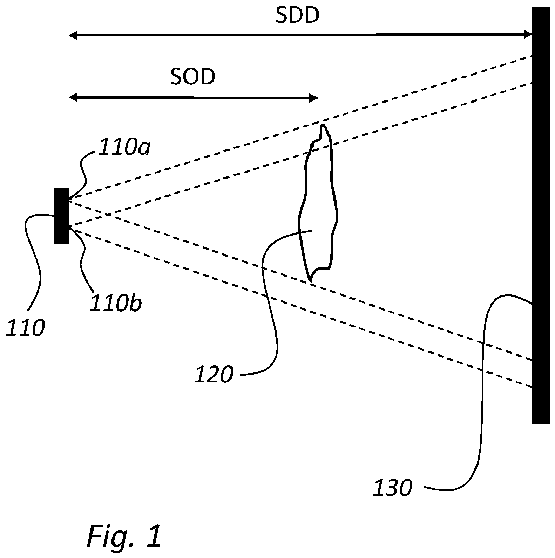

In projection radiography, an image is created by capturing X-ray radiation that has passed through and to varying degrees been absorbed by an object. Fig. 1 schematically shows a set-up illustrating the principles of the present invention. An electron beam (not shown in Fig. 1) is incident upon a target 110 to generate X-ray radiation, and after having passed a sample 120 located in a sample position region, the X-ray radiation is detected using an X-ray detector array 130. The region where the electron beam interacts with the target to produce X-ray radiation can be referred to as an X-ray spot and the size of this spot can be referred to as the spot size of the X-ray source. The image captured by the X-ray detector array is geometrically magnified by a factor M

$$M = \frac{SDD}{SOD}$$

where $SDD$ is the source-to-detector distance and $SOD$ is the source-to-object distance. In order to achieve maximum sharpness in the captured image, the spot size of the X-ray source should be as small as possible. Any finite size X-ray source will result in an image un-sharpness given by

$$\text{un-sharpness} = \frac{d}{SOD}(SDD - SOD)$$

where d is the source size, e.g. the diameter of the X-ray spot.

In order to reach short exposure times with sufficient contrast, the total X-ray flux must be sufficiently high. The X-ray flux can be increased by increasing the power of the electron beam impinging on the target. However, increasing the electron beam power while maintaining the size of the electron beam spot will increase the power density delivered to the target. If the power density becomes too high, this may cause permanent damage to the target. Hence, in order to apply more electron beam power to the target and thereby increase the total X-ray flux, it has been required in the prior art to increase the source size. This, on the other hand, leads to reduced image sharpness as discussed above.

Hence, any attempt to increase the total photon flux by increasing the electron beam power and making the X-ray source spot size larger (to limit the peak power delivered to the target) will inevitably result in a reduced image sharpness.

To resolve these seemingly contradictory requirements, embodiments of the present invention use a movable electron beam that is alternately directed to at least a first 110a and a second 110b location on the target 110. From a thermal point of view, this will correspond to using a larger electron spot since the applied power will be distributed over a larger area of the target. However, the image sharpness will not necessarily be adversely affected since the spot size at each location does not need to be increased.

To preserve the high resolution available from a single spot (the spot at 110a or 110b as illustrated in Fig. 1), the detected photons should be associated with the particular location from which they originated, i.e. the detector 130 and the electron beam should be configured so that it can be determined if the X-ray radiation received by the X-ray detector 130 at any one instant originates from the first 110a or from the second 110b location. For an object 120 located in a region where radiation from the separate locations 110a, 110b overlap, a composite image of high sharpness can then be reconstructed in post processing.

Any target has some limit for how much power that can continuously be applied on a certain area without damaging the target. A higher power can, however, be applied intermittently provided that the time period during which such higher power is applied is short on a time scale for heating and cooling of the target. The average power applied to a particular location on the target will be the electron beam power (which can be assumed to be constant) times a duty cycle, i.e. the fraction of time that each location on the target is impacted by the electron beam. The total power that may be delivered to the target without causing damage may also limit the power that may be applied to each particular location. This limit may be increased e.g. by providing better cooling of the target.

As an example, consider a target that can handle a continuous thermal load of $P_0$ distributed over a circular spot of diameter $D$. Assuming that the electron beam is alternately deflected between two different locations with a duty cycle of 50% (i.e. the electron spot is directed at the respective location during 50% of the time) and ignoring the time required for moving the electron beam between the two locations, the electron beam power may be increased by a factor of 2. In this manner, the average power applied to the respective location will still be $P_0$, but the total power delivered to the target will be $P_0$ divided by the duty cycle which equals $2P_0$. This may be generalized to more than two locations. For a case where the electron beam alternates between n locations, the duty cycle will be $1/n$ and the maximum total power that can be delivered to the target becomes $nP_0$ (again assuming that the time required for moving the electron beam between locations can be neglected). To be able to achieve the maximum total power without damaging the target, the locations need to be sufficiently spaced apart so that power applied to one location does not contribute to the heating of an adjacent location. Again assuming that the maximum allowed power for spot size $D$ is $P_0$ one may consider the higher power $nP_0$ applied during a short time to be distributed over an effective area of size $D_{eff}$. By prescribing that the average power density should be the same it can be seen that $D_{eff}$ must scale as $\sqrt{n}$. Thus, the distance between adjacent location may be selected to be larger than $\sqrt{n}D$. In cases where the electron beam is moved over the target in a continuous fashion along a closed path, the duty cycle may be approximated as the electron beam spot diameter $D$ divided by the length of the path ($L$). Thus, the total power delivered to the target in such case will be $P_0L/D$.

To be able to apply higher electron beam power than what can be absorbed continuously by the target at a particular location, the time during which the higher power is applied should be shorter than some time limit. This time limit will depend on the type and characteristic of the target as discussed below.

Depending on the target material used and the power levels involved in various embodiments of the invention, the inventive scheme may introduce issues relating to target lifetime due to repeated cooling and heating of the target material. Such thermal cycling may in some situations give rise to thermally induced mechanical stress which in turn may result in permanent damage to the target. To avoid this, the electron beam can be alternated between different locations on the target at a sufficiently high rate (short time period) so as to emulate a thermal steady state in the target. A characteristic time scale for target heating is given by the thermal conductivity and the volumetric heat capacity of the target and the electron beam spot size. It can be assumed that the time scale for target heating depends on the following characteristics

| | | | |
|---|---|---|---|
| $C$ | target heat capacity per unit mass | [J/(kg·K)] |
| $\rho$ | target density | [kg/m$^3$] |
| $\kappa$ | target heat conductivity | [W/m·K] |
| $\delta$ | electron beam spot size | [m] |
| $h$ | electron penetration depth in target | [m] |
| $t$ | thickness of a transmission target | [m] |

The relevant time scale may be deduced by estimating the amount of energy deposited in the target by the electron beam and the thermal power conducted away by the target. It can be noted that, while the power of the electron beam will influence the temperature levels reached in the target, the characteristic time scale only depends on target properties and the geometry of the electron beam. For a transmission target (a target of a kind where the electron beam impinges on one side and X-ray radiation is collected from an opposite side, i.e. in "transmission"), of a thickness $t$, the volume in which heat is deposited may be approximated by a cylinder with a (not necessarily circular) cross-sectional area defined by the electron beam spot size and a height defined by the target thickness. For a non-circular spot the electron beam spot size $\delta$ may considered as the geometrical average of the spot size. The amount of energy deposited in the target per degree temperature increase may then, ignoring numerical factors, be written as

$$C\rho\delta^2 t$$

$$(1)$$

An increase in temperature within the volume impacted by the electron beam will drive a heat flow from this volume through the area, $A$, of the envelope surface of the cylinder (for a transmission target heat conduction in the direction of the electron beam may be neglected). The heat needs to travel some average distance, $l$, to escape from the cylinder; this distance is in an approximation determined by the electron beam spot size. The power conducted away from the volume impacted by the electron beam per degree temperature increase may then, ignoring numerical factors, be written as

$$\kappa\frac{A}{l} = \kappa\frac{\delta t}{\delta} = \kappa t$$

$$(2)$$

By dividing the deposited energy per degree temperature increase according to expression (1) with the dissipated power per degree temperature increase according to expression (2), a characteristic time scale $\tau$ may be written as

$$\tau = \frac{C\rho}{\kappa}\delta^2$$

$$(3)$$

In practice, a transmission target is often comprised of a thin film of X-ray generating material, e.g. tungsten, provided on a substrate selected for its thermal and mechanical properties, e.g. diamond. In this case there will be heat transfer also through the end surface of the cylinder impacted by the electron beam. This contribution to the heat transport will depend on the heat transfer from the target material to the substrate material and on the thermal conductivity of the substrate material. Since the substrate is arranged to improve the thermal conductivity, more power will be dissipated per degree temperature increase and the characteristic time scale will thus be shorter than for a similar target provided without a substrate. The estimate provided above can thus be viewed as an upper limit on the characteristic time scale for a transmission target.

For a reflection target (a target of a kind where the electron beam impinges on one side of the target and X-ray radiation is collected from the same side of the target, i.e. in "reflection"), with a finite electron penetration depth $h$, the volume in which energy from the electron beam is deposited may be approximated by a cylinder with a base area determined by the electron beam spot size and a height given by the penetration depth. Similar to expression (1) above, the energy per degree temperature increase may thus, neglecting any numerical factors, be written as

$$C\rho\delta^2 h$$

$$(4)$$

The power driven from this cylinder will go through an area, $A$, defined by the envelope surface of the cylinder as well as its end surface. The average distance, $l$, the heat must travel is determined by both the electron beam spot size and the electron penetration depth. The two lengths may be seen as parallel paths so the inverse of the average distance is the sum of the inverse of the two lengths. The dissipated power per degree temperature increase may thus, ignoring any numerical factors, be written as

$$\kappa\frac{A}{l} = \kappa(\delta^2 + \delta h)\left(\frac{1}{\delta} + \frac{1}{h}\right)$$

$$(5)$$

As for the case of a transmission target, a characteristic time scale may be obtained by the dividing the deposited energy per degree temperature increase according to expression (4) by the dissipated power per degree temperature increase according to expression (5).

$$\tau = \frac{\rho C}{\kappa}\frac{\delta^2 h}{(\delta^2 + \delta h)\left(\frac{1}{\delta} + \frac{1}{h}\right)} = \frac{\rho C}{\kappa}\frac{\delta^2 h^2}{(\delta + h)^2} = \frac{\rho C}{\kappa}\delta^2 h S$$

$$(6)$$

where $S$ is a geometrical factor of dimension inverse length given by

$$S = \frac{h}{(\delta + h)^2}$$

$$(7)$$

For large electron spots where it can be assumed that $\delta \gg h$, $S$ reduces to $h/\delta^2$ and the time scale is then mainly determined by the penetration depth

$$\tau = \frac{\rho C}{\kappa}h^2$$

$$(8)$$

For tightly focused electron spots where it can be assumed that $h \gg \delta$, $S$ reduces to $1/h$ and the time scale is then mainly determined by the electron spot size

$$\tau = \frac{\rho C}{\kappa}\delta^2$$

$$(9)$$

In this latter case, heat conduction through the end surface of the heated cylinder may be neglected and the situation becomes the same as for a transmission target, and thus the characteristic time scale in this limit is the same as for a transmission target, see expression (3) above.

As a numerical example, assuming the target is made from tungsten having $C$=130 J/(kg K), $\rho$=19300 kg/m$^3$, $\kappa$=173 W/(m K) and assuming that the electron spot size and the penetration depth are both 5 $\mu$m, the expression (6) above gives a characteristic time scale of about 90 ns. For a larger electron beam spot of 20 $\mu$m and the same electron penetration depth of 5 $\mu$m, the characteristic time scale becomes about 230 ns.

For a liquid jet target the situation is a bit different. The dominating contribution to cooling of the target at the electron beam spot is the flow of target material. The characteristic time scale for this process may written as

$$\tau = \frac{\delta}{v_{jet}}$$

$$(10)$$

where $\delta$ is a spot size of the electron beam at the liquid jet target along a travel direction of the liquid jet and $v_{jet}$ is a travel velocity of the liquid jet.

A rotating anode with realistic parameters may in this context be considered as a stationary reflective solid target. To achieve target velocities of the same order as typically used for liquid jets (the order of hundreds of m/s) would require unrealistically large and/or fast spinning anodes. In such applications a line focus is typically used with the longer dimension of the focus perpendicular to the rotation direction. Within the present inventive concept a circular spot moved along a direction perpendicular the rotation direction may be used instead. This may furthermore enable a more advantageous take off angle than normally used with rotating anodes.

To be able to intermittently apply a higher power to a location on the target than a power that the target can withstand under prolonged exposure, the period of continuous exposure of any one location on the target (before the electron beam is moved away from that location) is preferably less than 10 times the characteristic time scale derived above, more preferably less than 5 times the characteristic time scale.

To protect the target from damage due to thermal cycling, it is also preferred that the time period for switching the electron beam between the different locations on the target is preferably less than 10 times the characteristic time scale derived above, more preferably less than 5 times the characteristic time scale. In other words, the time elapsed between consecutive periods during which the electron beam is directed to each of the locations on the target, respectively, is preferably shorter than 10 times, more preferably less than 5 times, the characteristic time scale.

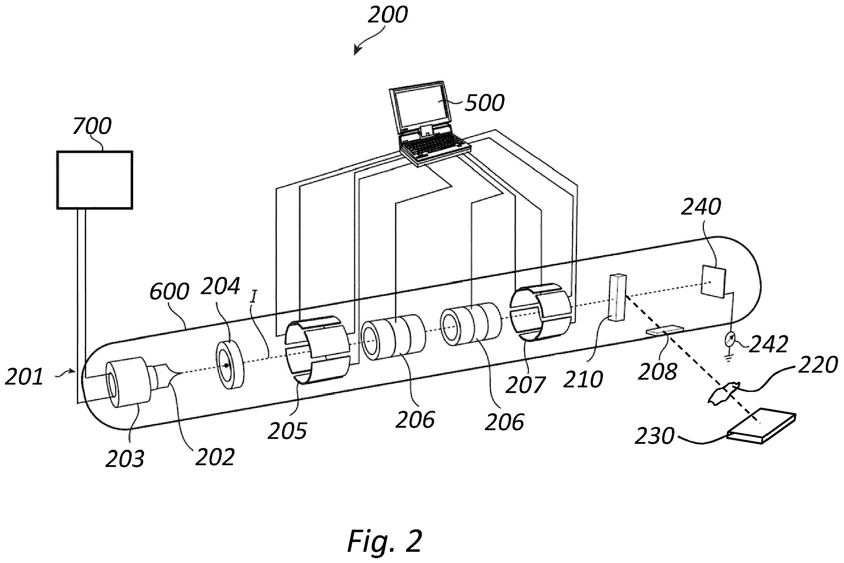

An X-ray imaging system 200 according to the present invention is schematically shown in Fig. 2. It comprises a detector 230 for recording images, e.g. of an object or a sample 220, created from X-ray radiation from the respective electron beam spot location (see 110a and 110b in Fig. 1). The system 200 comprises an X-ray target 210, and an electron source 201 for generating an electron beam I. The electron source 201 generally comprises a cathode 202 which is powered by a voltage supply 700 and includes an electron source 203, e.g., a thermionic, thermal-field or cold-field charged-particle source. The electron beam / from the electron source 201 may be accelerated towards an accelerating aperture 204, at which point the beam / enters a region with electron optics which may comprise an arrangement of aligning plates 205, lenses 206 and an arrangement of deflection plates 207. Variable properties of the aligning plates 205, deflection plates 207 and lenses 206 may be controllable by signals provided by a controller 500. As illustrated, the deflection and aligning means 207, 205 are operable to accelerate the electron beam / in at least two transversal directions.

The various components and parts mentioned above may be located inside a housing 600, with possible exceptions for the voltage supply 700 and the controller 500, which may be located outside the housing 600 as shown in the drawing.

Downstream of the electron optics, the electron beam / may impinge upon the X-ray target 210 to generate X-ray radiation. The X-ray radiation generated by interaction between the electron beam / and the target 210 may be led out from the housing 600, via e.g. an X-ray window 208, in a direction generally towards the sample 220 and the detector array 230.

The system may also optionally comprise a sensor arrangement 240 for measuring/detecting electrons downstream from the target 210. Such sensor may e.g. be a conductive plate connected to ground via an ammeter 242, which provides an approximate measure of the total current carried by the electron beam / downstream of the target 210. It is understood that the controller 500 has access to the actual signal from the ammeter 242.

The size of the detector 230 may determine the available size of the sample position region. Since the invention relies on imaging a sample with X-ray radiation originating from different locations of the target 210, the volume where the X-ray radiation and the field of view of the detector overlap defines the usable sample position region. To avoid limiting image quality by the detector, the pixel size should be small compared the un-sharpness that is necessarily introduced by the finite size of the X-ray source (i.e. spot). However, the required pixel resolution may be determined from the characteristic length scale of the objects intended to be inspected and the magnification of the X-ray imaging system. To sort the incoming X-ray radiation in accordance with its point of origin, the detector must be able to record a timestamp for each detection event or bin together detection events that occur within some time window or set of time windows. The time resolution of the detector should preferably be short compared to the time during which the electron beam is located at each of the respective spot sites. An example of detector technology that may be advantageous is the Timepix3 chip developed within the Medipix collaboration hosted by CERN. In general, detectors that provide for processing capability associated with each pixel may be used to an advantage in the present invention.

Provided the detector creates a timestamp for each detection event, the detection events may be associated with an electron beam position provided these are known at the corresponding times. The electron beam motion may, for example, be pre-defined and known to the post-processing system. The first detection event may then be regarded as starting time of the electron beam motion pattern and the detection events may be mapped onto that pattern. Another embodiment may utilize a trigger signal provided by the X-ray source or the detector marking the start of the sequence. In yet another embodiment the X-ray source generates a protocol of electron beam positions as a function of time that is sent to the post-processing software together with the recordings from the detector. The post-processing software may then sort detection events to electron beam positions based on the times recorded by the X-ray source and the detector respectively. This requires that the X-ray source and the detector measure time on a common scale.

Creation of a high-resolution high-contrast image from two images obtained for two locations of the X-ray spot may be done by translating one of the images with respect to the other by an amount

$$\Delta = b\left(\frac{SDD}{SOD} - 1\right)$$

(11)

where b is the distance between the electron beam spot locations on the target. The expression for $\Delta$ may be obtained from basic geometry. After the translation, the two images may be added together. The skilled person realizes that adding two images together may be performed in a number of ways and may also include filtering to improve the signal to noise ratio. One example is to sum each individual pixel and divide the results by two. In this way the images are given equal weight and the pixel values are kept within the dynamical range of the original images.

Embodiments where the electron beam moves over the target in a continuous fashion, not necessarily stopping at a particular location, are also included within the inventive concept. An example of such an embodiment comprises an electron beam that is continuously moved back and forth with a pre-determined frequency, e.g. by applying a triangular wave driving signal to a deflection plate. Collecting photons emitted from a moving spot will mean that motion blurring may be introduced, similar to the un-sharpness caused by a finite size X-ray spot as discussed above. The speed of motion of the electron beam should thus be set so that motion blur introduced during the time between readouts from the detector may be considered negligible. In an embodiment where the electron beam is moved between different locations at a constant frequency, images collected at the same frequency will be generated by X-ray radiation emitted from the same locations. Thus, by shifting the positions of these images in post-processing in accordance with the prescribed motion, an image with high resolution and high contrast may be obtained. If the exact timing is not known, i.e. the post processing system does not have access to the electron beam location at a particular time, different assumptions may be tried until the image contrast cannot be further improved. This may be viewed as finding the phase of the electron beam motion when the frequency and amplitude are known.

As an example, consider a set-up for imaging a millimeter-sized object, such as an insect or a flower bud. A triangular wave driving signal applied to a deflection plate may be configured to move the electron beam back and forth between two locations on the target 1 mm apart. The imaging optics may then suitably provide a magnification of about 3 in order to give a decent resolution for propagation-based phase contrast imaging on the above-mentioned Timepix2 chip with 55 $\mu$m pixels. A suitable source-to-detector distance may in this case be about 2 m. An electrostatic deflection driven by the triangular driving signal may be set to a frequency of 1 MHz and an amplitude that provides the 1 mm separation between the extremal points of the electron beam on the target, such that the spot moves by 3 $\mu$m in the 1.5 ns time precision of the Timepix3 detector. The detected X-ray radiation is then binned in 1 ns larges bins according to their respective time stamps modulo 1 $\mu$s. Each bin will then give one image when including the pixel coordinate datum. The images can then be combined in post processing by shifting (i.e. translating) the images following the original triangular driving signal with different phases to find which phase provides the highest contrast.

At least two images taken from different directions may be used to obtain a partial tomographic reconstruction of a sample. Techniques developed for tomosynthesis and computed laminography may be employed since these have been developed for cases where only a limited number of projections are available. The skilled person will realize that it may be advantageous to combine translation and addition of the images as discussed above with different tomographic techniques to extract as much information as possible from the obtained images.

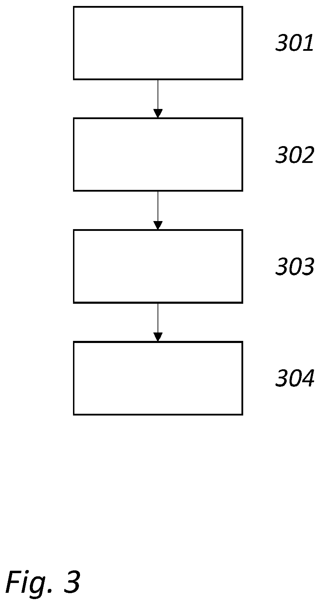

Fig 3. shows schematically a method according to the present invention. X-ray radiation is generated 301 by directing an electron beam onto a target, wherein the electron beam is alternately directed to at least a first and a second location on the target, thereby alternately generating X-ray radiation at the first and the second location. To prevent overheating of the target, the electron beam should not be kept at the same location for more than a prescribed time limit. Preferably, this time limit is less than 10 times, in particular less than 5 times, the characteristic time scale $\tau$ given by expressions (6) and (7) above. To further prevent target damage due to thermal cycling the electron beam should alternate between locations at a sufficiently high rate, i.e. the time period of the alternating motion should be below a time limit. Preferably, the electron beam is alternately directed to the first location and the second location on the target with a time period less than 10 times, in particular less than 5 times, the characteristic time scale $\tau$ given by expressions (6) and (7) above. In some embodiments, the electron beam is blanked, i.e. blocked, during a switch between the first location and the second location, such that no X-ray radiation is generated from regions outside the first location and the second location. In other embodiments, the electron beam continuously impacts the target as it is swept between the first location and the second location. While this disclosure takes as an example that there are two primary locations from which X-ray radiation is generated, it will be apparent that more than two locations can be used for generating the X-ray radiation, and that X-ray radiation will be generated from a plurality of locations in embodiments where the electron beam is swept across the target. As an example, the electron beam can be directed onto the target in accordance with a predetermined pattern, which can be used for subsequent creation of an image from detected X-ray radiation.

The generated X-ray radiation is directed 302 to a sample position region, which is where a sample to be studied can be placed. The sample position region is located in a region where X-ray radiation generated at the first location overlaps with X-ray radiation generated at the second location. X-ray radiation that has passed through the sample position region is then detected 303 using an X-ray detector array. The detection of X-ray radiation that has passed through the sample position region is correlated 304 with the direction of the electron beam, and an image is created based on the X-ray radiation originating from the first location and from the second location. In embodiments where the electron beam is directed onto the target in accordance with a predetermined pattern, such correlation between the direction of the electron beam and the detected X-ray radiation can be based on the predetermined pattern as described above. In some embodiments there is created a partial tomographic reconstruction based on the X-ray radiation originating from the first location and from the second location.

Conclusion

There is disclosed an X-ray imaging system, comprising a target; an electron beam source configured to provide an electron beam for interaction with the target to generate X-ray radiation; electron optics configured to alternately direct the electron beam to at least a first and a second location on the target; an X-ray detector array configured to receive X-ray radiation generated at the first and second locations on the target; a sample position region for receiving a sample to be exposed to generated X-ray radiation, the sample position region being located in a region where X-ray radiation generated at the first location overlaps with X-ray radiation generated at the second location; and a processing unit coupled to the X-ray detector array, the processing unit being configured to create an image of a sample, positioned in the sample position region, based on the X-ray radiation originating from the first location and from the second location. In embodiments of the invention, the electron beam is moved such that the exposure time for the X-ray radiation generated each time the electron beam is directed to a particular location is sufficiently short. In further embodiments, the electron beam is moved between the locations at a sufficiently short time period such that thermal cycling, and thereby thermally induced mechanical stress, is reduced. A corresponding method is also disclosed.

**Claims**

1.  An X-ray imaging system, comprising
    a target;
    an electron beam source configured to provide an electron beam for interaction with the target to generate X-ray radiation;
    electron optics configured to alternately direct the electron beam to at least a first and a second location on the target;
    an X-ray detector array configured to receive X-ray radiation generated at the first and second locations on the target;
    a sample position region for receiving a sample to be exposed to generated X-ray radiation, the sample position region being located in a region where X-ray radiation generated at the first location overlaps with X-ray radiation generated at the second location; and
    a processing unit coupled to the X-ray detector array, the processing unit being configured to create an image of a sample, positioned in the sample position region, based on the X-ray radiation originating from the first location and from the second location;

wherein the x-ray detector array and the electron beam source are configured such that it can be determined if the x-ray radiation received by the x-ray detector at any one instant originates from the first location or from the second location on the target.

2. The system of claim 1, wherein the electron optics is configured to alternately direct the electron beam to the at least first location and second location on the target such that a period of continuous exposure of any one location has a duration that is shorter than a time limit.

3. The system of claim 2, wherein the electron optics is configured to alternately direct the electron beam to the at least first location and second location on the target at a rate such that a time elapsed between consecutive periods during which the electron beam is directed to each of the first and the second location on the target, respectively, is shorter than said time limit.

4. The system of claim 2 or claim 3, wherein the target is a solid reflection target and the time limit is 10 times, preferably 5 times, a characteristic time scale, $\tau$, given by

$$\tau = \frac{\rho C}{\kappa} \delta^2 h \cdot S$$

where S is given by

$$S = \frac{h}{(\delta + h)^2}$$

and where $\rho$ is a density of the target, $C$ is a heat capacity per unit mass of the target, $\kappa$ is a heat conductivity of the target, $\delta$ is a spot diameter of the electron beam at the target, and $h$ is a penetration depth of electrons into the target.

5. The system of claim 2 or claim 3, wherein the target is a transmission target and the time limit is 10 times, preferably 5 times, a characteristic time scale, $\tau$, given by

$$\tau = \frac{\rho C}{\kappa} \delta^2$$

where $\rho$ is a density of the target, $C$ is a heat capacity per unit mass of the target, $\kappa$ is a heat conductivity of the target, and $\delta$ is a spot size of the electron beam at the target.

6. The system of claim 2 or claim 3, wherein the target comprises at least one liquid jet and the time limit is 10 times, preferably 5 times, a characteristic time scale, $\tau$, given by

$$\tau = \frac{\delta}{v_{jet}}$$

where $\delta$ is a spot size of the electron beam at the target along a travel direction of the at least one liquid jet and $v_{jet}$ is a travel velocity of the at least one liquid jet.

7. The system of any one of the preceding claims, wherein the electron beam source is configured to blank the electron beam during a switch between the first location and the second location on the target, such that no X-ray radiation is generated from regions of the target outside of the first location and the second location.

8. A method for X-ray imaging, comprising:

generating X-ray radiation by directing an electron beam onto a target, wherein the electron beam is alternately directed to at least a first and a second location on the target thereby alternately generating X-ray radiation at the first and the second location;

directing the generated X-ray radiation to a sample position region, wherein the sample position region is located in a region where X-ray radiation generated at the first location overlaps with X-ray radiation generated at the second location; and

detecting, using an X-ray detector array, X-ray radiation that has passed through the sample position region; and correlating the direction of the electron beam with the detection of X-ray radiation that has passed through the sample position region to determine if the X-ray radiation received by the X-ray detector array at any one instant originates from the first location or from the second location, and creating an image based on the X-ray radiation originating from the first location and from the second location.

9. The method of claim 8, wherein the electron beam is alternately directed to the at least first location and second location on the target such that a period of continuous exposure of any one location has a duration that is shorter than a time limit.

10. The method of claim 9, wherein the electron beam is alternately directed to the at least first location and second location on the target at a rate such that a time elapsed between consecutive periods during which the electron beam is directed to each of the first and the second location on the target, respectively, is shorter than said time limit.

11. The method of claim 9 or claim 10, wherein the target is a solid reflection target and the time limit is 10 times, preferably 5 times, a characteristic time scale $\tau$, given by

$$\tau = \frac{\rho C}{\kappa} \delta^2 h \cdot S$$

where $S$ is given by

$$S = \frac{h}{(\delta + h)^2}$$

and where $\rho$ is a density of the target, $C$ is a heat capacity per unit mass of the target, $\kappa$ is a heat conductivity of the target, $\delta$ is a spot diameter of the electron beam at the target, and $h$ is a penetration depth of electrons into the target.

12. The method of claim 9 or claim 10, wherein the target is a transmission target and the time limit is 10 times, preferably 5 times, a characteristic time scale, $\tau$, given by

$$\tau = \frac{\rho C}{\kappa} \delta^2$$

where $\rho$ is a density of the target, $C$ is a heat capacity per unit mass of the target, $\kappa$ is a heat conductivity of the target, and $\delta$ is a spot size of the electron beam at the target.

13. The method of claim 9 or claim 10, wherein the target comprises at least one liquid jet and wherein the time limit is 10 times, preferably 5 times, a characteristic time scale, $\tau$, given by

$$\tau = \frac{\delta}{v_{jet}}$$

where $\delta$ is a spot size of the electron beam at the target along a travel direction of the at least one liquid jet and $v_{jet}$ is a travel velocity of the at least one liquid jet.

**14.** The method of any one of claims 8-13, further comprising blanking the electron beam during a switch between the first location and the second location on the target, such that no X-ray radiation is generated from regions of the target outside of the first location and the second location.

**15.** The method of any one of claims 8-14, wherein the electron beam is directed onto the target in accordance with a predetermined pattern, and wherein the image is created based on the predetermined pattern.

SDD

SOD

110a

110

110b

120

130

*Fig. 1*

Fig. 2

EP 3 906 856 A1

301

302

303

304

*Fig. 3*

Europäisches
Patentamt

European
Patent Office

Office européen
des brevets

# EUROPEAN SEARCH REPORT

Application Number

EP 20 17 3258

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 183 139 B1 (SOLOMON EDWARD G [US] ET AL) 6 February 2001 (2001-02-06) | 1-6, 8-13,15 | INV. A61B6/00 H01J35/08 |
| Y | * column 3 - column 7; figures 1,4 * ----- | 7,14 | |
| Y | US 2015/098548 A1 (BATHE CHRISTOPH HELMUT [DE] ET AL) 9 April 2015 (2015-04-09) * paragraph [0021] * ----- | 7,14 | |

TECHNICAL FIELDS
SEARCHED      (IPC)

A61B
H01J

The present search report has been drawn up for all claims

| Place of search | Date of completion of the search | Examiner |
|---|---|---|
| Munich | 30 September 2020 | Koprinarov, Ivaylo |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**EP 3 906 856 A1**

## ANNEX TO THE EUROPEAN SEARCH REPORT
## ON EUROPEAN PATENT APPLICATION NO.

EP 20 17 3258

30-09-2020

| Patent document cited in search report | | Publication date | Patent family member(s) | | Publication date |
|---|---|---|---|---|---|
| US 6183139 | B1 | 06-02-2001 | AU | 1441800 A | 26-04-2000 |
| | | | EP | 1119870 A1 | 01-08-2001 |
| | | | JP | 4562916 B2 | 13-10-2010 |
| | | | JP | 2002527857 A | 27-08-2002 |
| | | | US | 6183139 B1 | 06-02-2001 |
| | | | WO | 0021114 A1 | 13-04-2000 |
| US 2015098548 | A1 | 09-04-2015 | CN | 104335318 A | 04-02-2015 |
| | | | EP | 2852965 A1 | 01-04-2015 |
| | | | JP | 2015522910 A | 06-08-2015 |
| | | | RU | 2014151775 A | 20-07-2016 |
| | | | US | 2015098548 A1 | 09-04-2015 |
| | | | WO | 2013175370 A1 | 28-11-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5835561 A

**Non-patent literature cited in the description**

- Scanning-beam digital X-ray (SBDX) system for cardiac angiography. *Proc. SPIE 3659, Medical Imaging,* 1999
- **SOLOMON.** *Physics of Medical Imaging,* 28 May 1999
- **HARMON.** Motionless electromagnetic phase stepping versus mechanical phase stepping in x-ray phase-contrast imaging with a compact source. *Phys. Med. Biol.,* 2015, vol. 60, 3031-3043